# EUROPEAN PATENT SPECIFICATION

(11) **EP 1 965 699 B1**
(45) Date of publication and mention of the grant of the patent: **28.06.2017**
(21) Application number: 06851012.2
(22) Date of filing: 20.12.2006
(51) Int. Cl.: A61B 34/30, H04N 7/18

(54) **MEDICAL ROBOTIC SYSTEM PROVIDING THREE-DIMENSIONAL TELESTRATION**
MEDIZINISCHES ROBOTERSYSTEM FÜR DREIDIMENSIONALE TELESTRATION
SYSTÈME ROBOTIQUE MÉDICAL POUR TÉLÉ-ILLUSTRATION TRI-DIMENSIONNELLE

(30) Priority: 30.12.2005 US 322879
(43) Date of publication of application: 10.09.2008
(60) Divisional application: 16180658.3
(73) Proprietor: Intuitive Surgical Operations, Inc., Sunnyvale CA 94086 (US)
(72) Inventor: HASSER, Christopher, Los Altos, California 94024 (US); LARKIN, David, Menlo Park, California 94025 (US); MILLER, Brian, Lincoln, Nebraska 68512 (US); ZHANG, Guanghua, San Jose, California 95129 (US); NOWLAN, William, Los Altos, California 94024 (US)
(74) Representative: MacDougall, Alan John Shaw
(86) International application number: PCT/US2006/062381
(87) International publication number: WO 2007/120351

(56) References cited:
- WO-A1-01/29681
- US-A- 5 808 665
- US-A- 5 839 441
- US-A- 6 108 458
- US-A1- 2002 140 694
- US-B2- 6 856 324
- JOHN J. BAUER ET AL: "International Surgical Telementoring Using a Robotic Arm: Our Experience", TELEMEDICINE JOURNAL, vol. 6, no. 1, 9 July 2004 (2004-07-09), pages 25-31, XP055104819, ISSN: 1078-3024, DOI: 10.1089/107830200311824

## Description

### STATEMENT REGARDING FEDERALLY SPONSORED RESEARCH OR DEVELOPMENT

This invention was made with government support under contract no. 1 R41 EB004177-01 awarded by the National Institutes of Health. The government has certain rights in the invention.

### FIELD OF THE INVENTION

The present invention generally relates to minimally invasive robotic surgery systems and in particular, to a medical robotic system providing three-dimensional telestration.

### BACKGROUND OF THE INVENTION

Minimally invasive surgical methods such as laparoscopy and thoracoscopy can dramatically reduce morbidity, reduce acuity of care, speed recovery times, and lead to more satisfied patients. Surgeons performing conventional laparoscopy or thoracoscopy, however, face a steep learning curve and must cope with serious degradation of their ability to see and touch the operating field, as well as a dramatic reduction in their dexterity compared to open surgery.

Surgical telerobots can give surgeons high-fidelity three-dimensional (3D) vision and an intuitive articulated wrist at the end of the tool shaft, fundamentally improving surgeons' ability to sense and manipulate objects in the surgical field. Telerobots can also scale surgeons' hand motions down and eliminate tremor for more precise manipulation. These advances allow surgeons to accomplish the previously impossible, such as totally endoscopic coronary artery bypass surgery, and speed adoption of difficult procedures such as totally endoscopic radical prostatectomies.

The emergence of minimally invasive surgery (MIS) as the standard approach for a wide variety of surgical procedures has increased the importance of laparoscopic skill acquisition for surgeons-in-training and for practicing surgeons. The current surgical training model does not provide adequate experience in advanced MIS, and the learning curve for complex MIS procedures can lead to increased complications for inexperienced surgeons.

The challenge of training surgical residents in advanced laparoscopy has become more difficult as MIS procedures have become increasingly complex. Minimally invasive surgical education requires the development of a new set of surgical manipulation and visualization skills. To meet this need, the current gold standard is a dedicated post-residency MIS fellowship. Several strategies such as inanimate laboratories and simulation training have also been developed to increase the exposure of residents to advanced laparoscopic surgery during initial training, with varying success rates.

An even greater challenge faces already-practicing surgeons interested in performing advanced minimally invasive surgery. Strong patient demand for MIS procedures as well as ongoing shift in surgical standard of care toward less invasive approaches provides motivation; however, these surgeons often have difficulty translating their open or basic MIS skills to advanced MIS procedures, leading to unsatisfactory surgical outcomes and increased complication rates.

The current training paradigm for practicing surgeons has centered on procedure-specific short courses with very limited hands-on experience in an inanimate or animal laboratory. Such strategies fall far short of disseminating a proper knowledge and experience base and provide essentially no experience in actual surgery on humans. Students will frequently require the presence of their surgical mentors at a number of initial procedures. At least one study has demonstrated that common laparoscopic training courses are insufficient to make a surgeon proficient, and a single proctored session by a visiting mentor may not be sufficient.

Conventional mentoring demands the physical presence of an experienced surgeon. For many new procedures, very few surgeons have acquired enough experience to proctor or mentor a case. This increases the demand placed on that small group of surgeons. Traveling to mentor cases takes time away from the mentor's practice and personal life, and has an expense borne by the learning surgeon and the patient.

Telestration (shortened from "tele-illustration"), where the mentor is able to create illustrations overlayed on the student's two-dimensional surgical view, has been demonstrated to be an effective learning tool. Telestration offers a method of mentoring which can be more explicit than verbal communication and less intrusive than mechanical demonstration, as the surgeon in training may remain at the helm. Telestration allows the mentor to provide clear and useful visual cues to the learning surgeon in the same room, or over a distance. Telestration has the potential to improve the accessibility of robotic surgery training opportunities, increasing the adoption rate for robotically assisted surgery.

One example of a robotic surgical system is the da Vinci^{®} Surgical System of Intuitive Surgical, Inc., Sunnyvale, California. The da Vinci^{®} Surgical System can be used for a wide variety of surgical procedures such as mitral valve repair, Nissen Fundoplication for the treatment of GERD disease, gastic bypass surgery for obesity, radical prostatectormy (da Vinci^{®} Prostatectomy) for the removal of the prostate, esophageal surgery, thymectomy for myasthenia gravis, and epicardial pacemaker leads for biventricular resynchronization.

A unique feature of the da Vinci^{®} Surgical System is its three-dimensional display which provides the operating surgeon with superior telepresence. The da Vinci^{®} Surgical System provides a right and left stereo image to the surgeon using two cathode ray tubes and a series of mirrors and objective lenses to create the illusion of a three-dimensional scene.

Telestration to the student in a truly binocular 3D laparoscopic environment represents a tremendous improvement over traditional 2D laparoscopic visualization in several critical ways. The learning curve required to translate a 2D operative image into a 3D mental anatomic model poses a significant challenge to the MIS novice and seasoned surgeon alike. While restoring native stereoscopic visualization in three dimensions greatly enhances surgical precision in general, there are numerous specific circumstances where such imaging is absolutely critical to successful patient outcomes. Technical maneuvers, such as control of vascular pedicles, nerve-sparing dissection, microvascular anastomosis, and cardiac dissection and anastomosis, require a detailed appreciation of every aspect of the respective anatomic structures.

One problem with telestrating on such a three-dimensional display, however, is that a mentor with a touch screen can only telestrate on a two-dimensional (2D) image, requiring the operating surgeon to touch a foot pedal, or other switching device, to switch from a 3D view to a 2D view to see the telestration. This gives the surgeon the benefit of telestration, but interrupts the flow of the procedure and removes the benefit of 3D vision.

To effectively understand communications from the mentor and apply them to the 3D operating field, the trainee should be able to perceive those communications in 3D, without breaking his or her flow to switch to a degraded 2D display to look at the mentor's drawings. Having the mentor's telestration occur live in the trainee's 3D display during surgery, rather than requiring the trainee to switch modes to 2D, will encourage more frequent and impromptu communications between the mentor and trainee. One option for providing 3D telestration would be to have the mentor use a 3D input device and a stereo display; however, the cost and logistics involved would severely limit the attractiveness and scalability of the solution.

The paper "International Surgical Telementoring Using a Robotic Arm: Our Experience, Telemedicine Journal, Volume 6, Number 1, 2000" discloses a personal computer-based system developed to immerse a surgical specialist into a distant operating room utilizing public telephone lines. The system incorporates standard video conferencing (VTC) hardware with live video telestration (white boarding) capability and remote robotic control of a laparoscope camera. Multiple laparoscopic surgical procedures were conducted to demonstrate the ability to telementor less experienced surgeons placed at remote locations in Austria, Thailand, Italy and Singapore. Telecommunications links were established to these remote locations that ranged in distance from approximately 4500 to 11,000 miles.

US 2002/140694 discloses a method for augmented reality guided instrument positioning. A graphics guide is determined for positioning an instrument that provides a substantially unobstructed view of the instrument during the positioning. The graphics guide is rendered in alignment with a predetermined path to be traversed during the positioning of the instrument.

### OBJECTS AND SUMMARY OF THE INVENTION

The present invention provides a method and a medical robotic system for 3D telestration as set out in the appended claims. Accordingly, one object of the present invention is to provide a method and a medical robotic system for telestrating on a 3D image of an anatomical structure that does not require a 3D input device and stereo display for the mentoring surgeon.

The present invention provides a method for telestrating on a 3D view of an anatomical structure generated from a pair of stereoscopic images of the anatomical structure, the method including the step of: receiving a telestration graphic input generated by a user interacting with a two-dimensional touch screen; characterized in that the 2D touch screen is displaying one of the pair of stereoscopic images of the anatomical structure, and in that the method includes the steps of: determining a corresponding telestration graphic input in the other of the pair of stereoscopic images by using a disparity map associated with the pair of stereoscopic images on selected points of the received telestration graphic input so that a 3D view of the telestration graphic input is displayable on a 3D display; and displaying the 3D view of the telestration graphic input as an overlay to the 3D view of the anatomical structure on the 3D display.

The present invention also provides a medical robotic system providing 3D telestration, the system comprising a stereoscopic endoscope configured to generate a pair of stereoscopic images of an anatomical structure, a 2D touch screen, a surgeon computer configured to receive a telestration graphic input generated form user interaction with the 2D touch screen, and a 3D display for displaying a 3D view of the anatomical structure generated from the pair of stereoscopic images of the anatomical structure, wherein the system is characterized in that the 2D touch screen is configured to receive one of the pair of stereoscopic images captured by the stereoscopic endoscope, and the surgeon computer is configured to: receive a telestration graphic input generated from user interaction with the 2D touch screen; determine a corresponding telestration graphic input for the other of the pair of stereoscopic images by using a disparity map associated with the pair of stereoscopic images on selected points of the received telestration graphic input so that a 3D view of the telestration graphic input is displayable on the 3D display; and cause the 3D view of the telestration graphic input to be displayed as an overlay to the 3D view of the anatomical structure on the 3D display.

Additional objects, features and advantages of the various aspects of the present invention will become apparent from the following description of its preferred embodiment, which description should be taken in conjunction with the accompanying drawings.

The scope of the invention is determined by the subject matter of appended claims 1 and 4. Further embodiments are disclosed in the dependent claims.

### BRIEF DESCRIPTION OF THE DRAWINGS

**FIG. 1** illustrates a top view of an operating room with a medical robotic system providing 3D telestration, utilizing aspects of the present invention.
**FIG. 2** illustrates a front perspective view of a master control station including a processor configured to utilize aspects of the present invention.
**FIG. 3** illustrates a block diagram of a medical robotic system providing 3D telestration, utilizing aspects of the present invention.
**FIG. 4** illustrates a block diagram of modules in and components coupled to the surgeon computer, utilizing aspects of the present invention.
**FIG. 5** illustrates a block diagram of modules in and components coupled to the expert computer, which are useful for practicing aspects of the present invention.
**FIG. 6** illustrates a flow diagram of a method for telestrating on a 3D image of an anatomical structure, utilizing aspects of the present invention.
**FIG. 7** illustrates a flow diagram of a method for overlaying a 3D telestration graphic input over a 3D anatomical structure, utilizing aspects of the present invention.
**FIG. 8** illustrates a flow diagram of a method for anatomy tracking and 3D telestration over a tracked anatomical structure, utilizing aspects of the present invention.
**FIG. 9** illustrates an example of epipolar geometry for a pair of stereoscopic images of a point in a 3D coordinate frame, which is useful for practicing aspects of the present invention.

### DETAILED DESCRIPTION OF THE PREFERRED EMBODIMENT

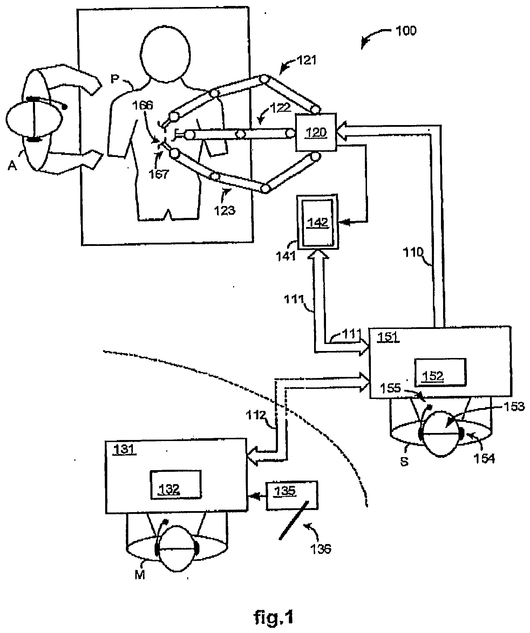

**FIG. 1** illustrates, as an example, a medical robotic system **100** providing three-dimensional telestration. In the example, an Operating Surgeon (S) is performing a minimally invasive surgical procedure on a Patient (P), and a Mentor Surgeon (M), who is an expert or at least more experienced in the minimally invasive surgical procedure, mentors or advises the Operating Surgeon (S) during the procedure. One or more Assistants (A) positioned at the Patient (P) site may also assist the Operating Surgeon (S) during the procedure.

The system **100** includes a surgeon master control station **151** (also referred to herein as the "surgeon console") operative by the Operating Surgeon (S), a slave cart **120** having three slave robotic mechanisms **121∼123**, and mentor master control station **131** (also referred to herein as the "mentor console") operative by the Mentor Surgeon (M). The mentor master control station **131** is shown separated from the surgeon master control station **151** by a dotted curved line since it may be either local to the surgeon master control station **151** (i.e., within the operating room environment) or remote from the surgeon master control station **151** (i.e., remote from the operating room environment).

The slave cart **120** is positioned alongside the Patient (P) so that surgery-related devices (such as surgery-related device **167**), which are coupled to distal ends of the slave robotic mechanisms **121∼123,** may be inserted through incisions (such as incision 166) in the Patient (P), and manipulated by the Operating Surgeon (S) at the surgeon master control station **151** to perform the minimally invasive surgical procedure on the Patient (P). Each of the slave robotic mechanisms **121∼123** preferably includes linkages that are coupled together and manipulated through motor controlled joints in a conventional manner.

Although only one slave cart **120** is shown as being used in this example, additional slave carts may be used as needed. Also, although three slave robotic mechanisms **121∼123** are shown on the cart **120,** more or less slave robotic mechanisms may be used per slave cart as needed. Additional details of a slave cart such as the slave cart **120** may be found in commonly owned U.S. Patent No. 6,837,883, "Arm Cart for Telerobotic Surgical System."

A stereoscopic endoscope is preferably one of the surgery-related devices coupled to the distal ends of the slave robotic mechanisms. Others of the surgery-related devices may be various tools with manipulatable end effectors for performing minimally invasive surgical procedures, such as clamps, graspers, scissors, staplers, and needle holders.

The number of surgery-related devices used at one time and consequently, the number of slave robotic mechanisms in the system **100** will generally depend on the diagnostic or surgical procedure and the space constraints within the operating room among other factors. If it is necessary to change one or more of the surgery-related devices being used during a procedure, one of the Assistants (A) may remove the surgery-related device that is no longer needed from the distal end of its slave robotic mechanism, and replace it with another surgery-related device from a tray of such devices in the operating room. Alternatively, a robotic mechanism may be provided for the Operating Surgeon (S) to execute tool exchanges using one of his or her master input devices.

To facilitate collaboration and/or mentoring of surgeons in minimally invasive surgical procedures, each of the participating surgeons has an associated display to view the surgical site, and a communication means such as a microphone and earphone set to communicate with other participating surgeons. Use of the stereoscopic endoscope in this case allows the generation and display of real-time, three-dimensional images of the surgical site.

More particularly, a 3D display **152** is coupled to or integrated into the surgeon master control station **151,** a 3D display **132** and a 2D touch screen **135** are coupled to or integrated into the mentor master control station **131,** and a 2D display **142** is provided on a vision cart **141,** so that the Operating Surgeon (S), Mentor Surgeon (M), and the one or more Assistants (A) may view the surgical site during the minimally invasive surgical procedure.

The communication means provided to each of the participants may include individual microphone and earphones (or speaker) components, or alternatively, individual headphone sets, such as headphone set **153** shown as being placed on the head of the Operating Surgeon (S), as part of a conventional audio system. Preferably a duplex audio communication system (microphone and speaker pair) is built into each surgeon's master control station. Alternatively, headsets may be used, including those using wireless communications to provide maximum comfort and freedom of movement to their users or those that may be connected through wires to their respective master control stations or slave cart, which are in turn, connected together through lines **110** and lines **112** for voice communications between the Operating Surgeon (S), Mentor Surgeon (M) and one or more Assistants (A).

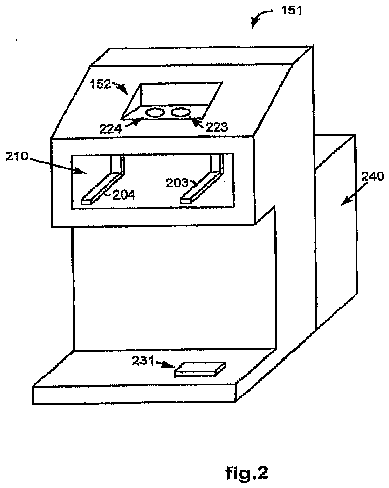

**FIG. 2** illustrates, as a simplified example, a front perspective view of the surgeon console or master control station **151.** Included in the surgeon console **151** is a 3D display **152** having right and left eye sockets, **223** and **224,** which are positioned so that a surgeon seated in front of the surgeon console **151** will look down through them to give the sensation that the surgical site viewed therein is at such a position. Also included are right and left master input devices, **203** and **204,** which are positioned within a recessed area **210** of the surgeon console **151** so that the surgeon has the sensation that he or she is directly manipulating associated instruments at the surgical site as viewed through the 3D display **152.** A processor **240** is coupled to or integrated into the surgeon console **151** to provide processing capability. A foot pedal **231** is also included in the surgeon console **151** to provide a switching capability, such as to turn telestration on and off, to hide a telestration and later recall it for display, or to switch between 3D and 2D views in the 3D display **151.** Alternatively, such switching capability may be implemented using a button on a telestration device, input device, or control console display, or it may be implemented by voice input.

Additional details of master control stations such as the surgeon master control station **151** may be found in commonly owned U.S. Patent No. 6,714,839, "Master Having Redundant Degrees of Freedom," and commonly owned U.S. Patent No. 6,659,939, "Cooperative Minimally Invasive Telesurgical System." The mentor master control station **131** may be similarly constructed as the surgeon console **151,** or alternatively, it may simply be a conventional personal computer with attached touch screen and digital pen for 2D viewing of the surgical site (as provided, for example, from the surgeon master control station **151**) and telestration on anatomical structures seen therein.

To perform a minimally invasive surgical procedure, the Operating Surgeon (S) may manipulate one or both of right and left master input devices, **203** and **204,** which in turn, causes associated slave robotic mechanisms, such as slave robotic mechanism **123,** to manipulate their respective surgery-related devices, such as surgical device **167,** through a minimally invasive incision, such as incision **166,** in the body of the Patient (P), while the Operating Surgeon (S) views the surgical site through his or her 3D display **152.**

Preferably, the master input devices will be movable in the same degrees of freedom as their associated surgery-related devices to provide the Operating Surgeon (S) with telepresence, or the perception that the master input devices are integral with their associated surgery-related devices, so that Operating Surgeon (S) has a strong sense of directly controlling them. To this end, position, force, and tactile feedback sensors are preferably employed that transmit position, force, and tactile sensations from the devices (or their respective slave robotic mechanisms) back to their associated master input devices so that the Operating Surgeon (S) may feel such with his or her hands as they operate the master input devices.

As previously described, to further enhance the telepresence experience, the 3D image of the surgical site (and anatomical structures seen therein), which is displayed on the 3D display **152** of the master control station **151** is oriented so that the Operating Surgeon (S) feels that he or she is actually looking directly down onto the operating site. To that end, an image of surgery-related devices that are being manipulated by the Operating Surgeon (S) appears to be located substantially where the his or her hands are located even though the observation points (i.e., the endoscope or viewing camera) may not be from the point of view of the image.

Additional details of a telepresence system and 3D display such as the medical robotic system **100** and 3D display **152** may be found in U.S. Patent No. 5,808,665, "Endoscopic Surgical Instrument and Method for Use," which is exclusively licensed by the assignee of the present invention; - - and commonly owned U.S. Patent No. 6,424,885, "Camera Referenced Control in a Minimally Invasive Surgical Apparatus."

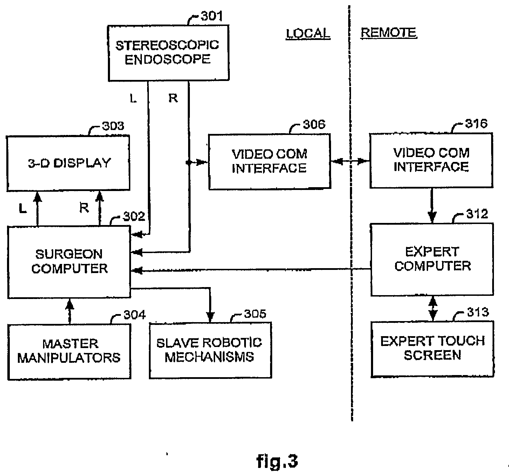

**FIG. 3** illustrates, as an example, a block diagram of parts of a medical robotic system providing 3D telestration. In this example, the Mentor Surgeon (M) is assumed to be remotely located (i.e., not in the operating room) while the Operating Surgeon (S) is locally located (i.e., in the operating room) along with the Patient (P).

Information for right (R) and left (L) camera views (or pairs of stereographic images), which have been captured by a stereoscopic endoscope (such as the surgery-related device coupled to slave robotic mechanism **122**) inserted in the surgical site within a patient, is received from stereoscopic endoscope **301** by a surgeon computer **302** (such as the processor **240** of the master control station **151**). At the same time, one camera view of each pair of stereographic images (such as, for example, the right camera view) is transmitted through video communication interfaces **306** and **316** to an expert or mentor computer **312** (such as a processor coupled to or integrated into the mentor master control station **131**). An example of a suitable video communication interface for such purpose is the Polycom VS4000 or VSX 7000e distributed by Polycom Inc. of Pleasanton, California. In addition to their use in transmitting the one camera view of an anatomical structure at the surgical site, the video communication interfaces **306** and **316** may also be used to communicate audio between the operating and expert surgeons respectively operating the surgeon computer **302** and the expert computer **312.**

The surgeon computer **302** processes the received information for the pairs of stereographic images, and provides them to 3D display **303** (such as 3D display **152** of the master control station **151)** for three-dimensional viewing by the Operating Surgeon (S). The Operating Surgeon (S) then manipulates master manipulators **304** (such as right and left master input devices **203** and **204**) to drive slave robotic mechanisms **305** (such as slave robotic mechanisms **121** and **123** of the slave cart **120**) and consequently, their attached surgery-related devices.

Meanwhile, the expert computer **312** processes the received camera view and provides it to touch screen **313** (such as touch screen **135** coupled to the mentor master control station **131**) for two-dimensional viewing by the Mentor Surgeon (M). An example of a suitable touch screen for such purpose is the Wacom Cintiq 15X distributed by Wacom Technology Corp. of Vancouver, Washington. The Mentor Surgeon (M) may then draw a telestration graphic on the surface of the touch screen **313** using a digital pen (such as the digital pen **136** coupled to the mentor master control station **131**). The telestration graphic may typically be a hand-drawn line, circle, arrow, or the like.

The expert computer **312** may then automatically transmit information of the telestration graphic input to the surgeon computer **302** real-time in parts via, for example, a TCP/IP connection, as the Mentor Surgeon (M) is drawing it, or it may transmit the entire telestration graphic input via the TCP/IP connection only after the Mentor Surgeon (M) has indicated that transmission should be made, for example, by clicking an appropriate button or switch on the touch screen **313** or its digital pen.

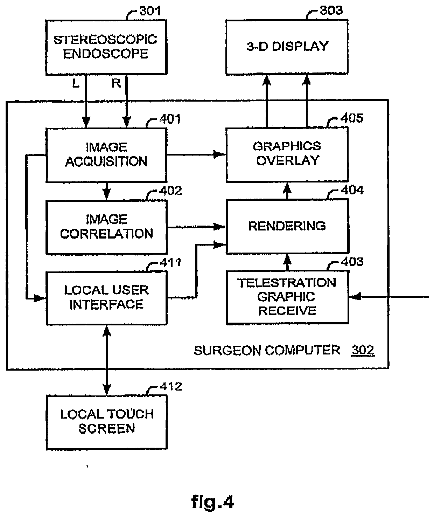

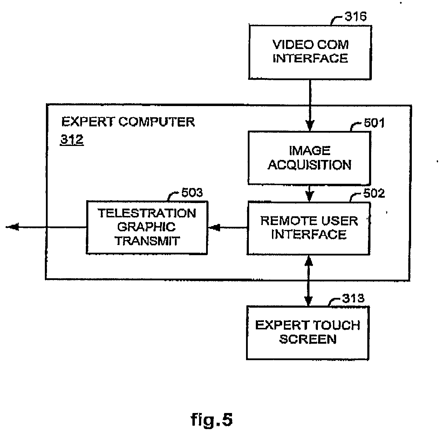

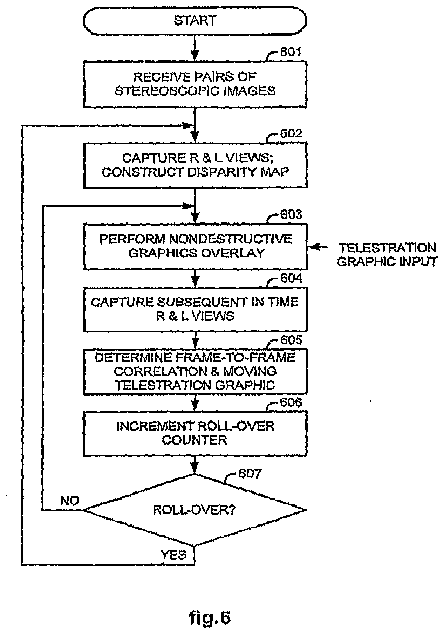

The surgeon computer **302** then processes the telestration graphic input received from the expert computer **312** so that a 3D view of the telestration graphic input may be displayed as an overlay to a 3D view of its corresponding anatomical structure in the 3D display **303,** according to the method described in reference to **FIG. 6****.** Additional details on the modules configured respectively in surgeon computer **302** and the expert computer **312** to perform their respective tasks as described herein, are further described below in reference to **FIGS. 4** and **5****.**

**FIG. 4** illustrates, as an example, a block diagram of modules providing the surgeon computer **302** with 3D telestration capability, and hardware components that interact with these modules of the surgeon computer **302;** and **FIG. 5** illustrates, as an example, a block diagram of modules providing the mentor or expert computer **312** with the capability to generate a telestration graphic input and transmit it to the surgeon computer **302** for 3D telestration of such graphic input, and hardware components that interact with these modules of the expert computer **312.**

Referring first to **FIG. 4****,** an image acquisition module **401,** such as a Matrox Orion frame grabber board distributed by Matrox Electronic Systems Ltd. of Canada, captures information of pairs of stereoscopic images from the stereoscopic endoscope **301,** such as in the left and right NTSC signals from the endoscope cameras, and provides that information to an image correlation module **402** which periodically generates or updates a disparity map using corresponding right and left camera views (or frames) captured by the image acquisition module **401.**

The output of the image acquisition module **401** may also be provided to a local user interface **411** which provides information for a selected one of the pairs of stereoscopic images to a local touch screen **412,** such as the Wacom Cintiq 15X, to be displayed in 2D on the touch screen **412.** A local expert or mentor surgeon may then telestrate on the touch screen **412** using a digital pen to generate a telestration graphic input which is provided to a rendering unit **404.**

The output of the image acquisition module **401** is also provided to a graphics overlay module **405,** which combines the captured pairs of stereoscopic images with a 3D telestration graphic input generated by the rendering unit **404,** and provides the combination to the 3D display 303 for three-dimensional viewing by an Operating Surgeon (S). The rendering unit **404** may receive a 2D telestration graphic input associated with one of the pairs of stereoscopic images from either a local mentor through the local user interface **411** or a remote mentor through a telestration graphic receive unit **403.**

Referring now to **FIG. 5****,** an image acquisition module **501,** such as the Matrox Orion frame grabber, captures information of the selected one of the pairs of stereoscopic images received by the video communications interface **316,** such as in the right NTSC signal from the endoscope cameras, and provides that information via a remote user interface 502 to a touch screen **313,** such as the Wacom Cintiq 15X, to be displayed in 2D on the touch screen **313.**

An expert or mentor surgeon may then telestrate on the touch screen **313** using a digital pen to generate a telestration graphic input which is provided via the remote user interface **502** to a telestration graphic transmit unit **503.** The telestration graphic transmit unit then transmits over TCP/IP, automatically in real-time or upon user command, the telestration graphic input as metadata, which may be in a selected graphics language format, to the telestration graphic receive unit **403** in the surgeon computer **302.**

**FIG. 6** illustrates a flow diagram of a method for telestrating on a 3D image of an anatomical structure, which is generally performed by modules in the surgeon computer **302** operating on information of pairs of stereoscopic images received from the stereoscopic endoscope **301.** Although it is assumed for the purposes of this example that telestration is being performed by a remote mentor surgeon (i.e., remote from the operating room environment), it is to be appreciated that the method is equally applicable to cases where telestration is being performed by a local mentor surgeon (i.e., in the operating room environment).

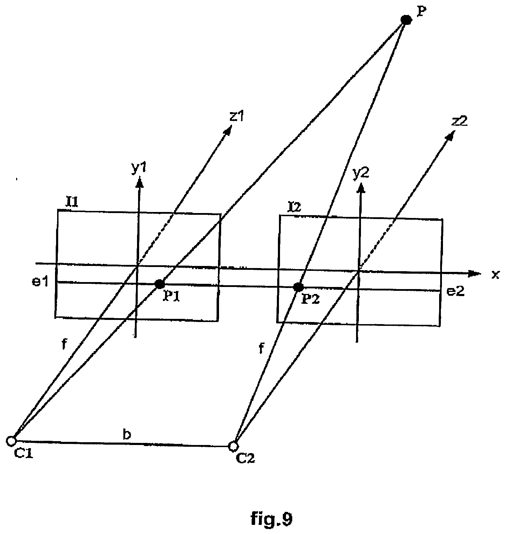

Prior to performing the method, the stereoscopic endoscope **301** is preferably fully calibrated for both its intrinsic and extrinsic parameters so that optical distortion is removed and the resultant perspective images are rectified into alignment. In particular, calibrating the stereoscopic endoscope **301** in this manner means that the disparity between correlated points in the left and right camera view images will lie along a horizontal epipolar line, as shown for example in **FIG. 9****,** which allows a one dimensional search with fewer chances for a false match thereby improving resolution and accuracy. This non-real-time camera calibration is generally performed using conventional techniques, such as with a Camera Calibration Toolbox for Matlab^{®} downloadable from the California Institute of Technology (Caltech) website.

In **601,** the image acquisition module **401** continuously receives information of a pair of stereoscopic images from a stereoscopic endoscope **301.** At the same time, the video communication unit **306** may continuously receive information for only a selected one of the pair of stereoscopic images (e.g., corresponding to one of the right and left cameras in the stereoscopic endoscope **301**) from the stereoscopic endoscope **301** for transmission to the remote expert touch screen **313.**

In **602,** the image acquisition module **401** captures or grabs a set of right and left camera views (i.e. right and left 2D frames) from the information received in **601,** and provides it to the image correlation module **402** which constructs a disparity map from the right and left camera views using an image correlation algorithm which is preferably fast enough for real-time operation and accurate enough to provide a 3D view of the surgical site which is suitable for minimally invasive surgical procedures. One example of such an image correlation algorithm is described in U.S. Patent No. 6,108,458 "Sparse Array Image Correlation" issued to Douglas P. Hart and assigned to the Massachusetts Institute of Technology.

In **603,** the rendering unit **404** first renders a 3D view of the telestration graphic input received from the remote mentor or local mentor. The graphics overlay module **405** then overlays the rendered 3D view of the telestration graphic input over a 3D view of the surgical site as provided by the stereo image pair received in **601.** Finally, the graphics overlay module **405** provides the 3D view of the surgical site with the overlayed 3D telestration graphic input so that the 3D display **303** may display them to the Operating Surgeon (S).

In **604,** the image acquisition module **401,** which continues to receive information of pairs of stereoscopic images from the stereoscopic endoscope **301**, captures or grabs another set of right and left camera views (i.e., right and left 2D frames) from information received subsequent in time from that previously captured.

In **605,** the right and left frames of the subsequently received information are correlated with their previously captured counterparts (i.e., the right frame captured at time t+1 is correlated with the right frame previously captured at time t+0, and the left frame captured at time t+1 is correlated with the left frame previously captured at time t+0) using an appropriate image correlation algorithm. By thus correlating the right and left frames with their previously captured counterparts, the movement of anatomic structures which are at the surgical site and in the camera view can be determined, and the 3D position of the telestration graphic input may be moved accordingly to track movement of the anatomic structure upon which it has been drawn. In addition, a confidence measure may be computed such as a correlation value, and the brightness of the displayed telestration graphic input may be proportional to the magnitude of the confidence measure.

In **606,** a rollover counter is incremented, and in **607,** the counter is checked to see if it has rolled over. If it hasn't, then the method loops back to repeat inner loop **603-607,** and if it has, the method loops back to repeat outer loop **602-607.** In this way, the generation of the disparity map in **602** may be performed less frequently than the anatomy tracking performed in **604-605.** For example, by properly selecting the clock frequency and the rollover value for the rollover counter, the inner loop **603-607** may be performed at a frequency of 30 Hz while the outer loop **602-607** is performed less frequently, such as at a rate of 1 Hz. Although a rollover counter is described as being used for this purpose, other conventional techniques for accomplishing the same or similar function may be used in its stead and are fully contemplated to be within the scope of the present invention.

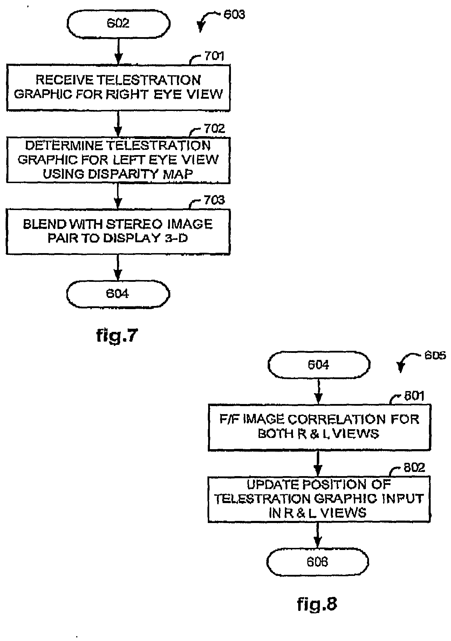

**FIG. 7** illustrates, as an example, a flow diagram detailing tasks executed by the rendering unit **404** and the graphics overlay module **405** in performing function **603** of the method described in reference to **FIG. 6****.** Although it is assumed for this example that a right camera view of the pair of stereographic images has been transmitted to a remote mentor surgeon for viewing and telestration, the following and other methods described herein are equally applicable to cases where the left camera view is transmitted instead.

In **701,** the rendering unit **404** receives information for a telestration graphic input corresponding to the right camera view of the pair of stereographic images from a remote mentor surgeon through the telestration graphic receive unit **403.** Since the received information preferably defines the telestration graphic input in a selected graphics language, the rendering unit **404** translates the received information as necessary to be compatible with the disparity map.

Preferably, the depth of the telestration graphic input is the same as the anatomic structure over which it is positioned in the right camera view. Thus, from the position of the received telestration graphic input corresponding to the right camera view, the depth of the telestration graphic input is readily determinable using the disparity map since the disparity map is directly associated with a depth map that can be determined non-real-time during the calibration process for the stereoscopic endoscope **301.**

In **702,** the rendering unit **404** then determines the telestration graphic input position in the left camera view which corresponds to the received telestration graphic input position in the right camera view. It does this by using the disparity map previously generated for the right and left camera views. In particular, for selected points of the received telestration graphic input corresponding to the right camera view, disparity values are read or otherwise determined from the disparity map at the locations of those points. The corresponding locations in the left camera view for those points are then determined by adjusting the locations in the right camera view by the disparity values.

In **703,** the graphics overlay module **405** overlays or blends the telestration graphic input positioned for the right camera view over or with the right camera view, and overlays or blends the telestration graphic input positioned for the left camera view over or with the left camera view. Preferably, both overlays are performed in a nondestructive manner so that underlying camera view information is preserved. The graphics overlay module **405** then provides the stereoscopic right and left camera view information with the overlayed 3D telestration graphic input to the 3D display **303** so that the Operating Surgeon (S) may view the surgical site with the 3D telestration graphic input properly positioned on the 3D anatomic structure. Optionally, the information may be provided to the 3D display **303** in such a fashion that the 3D telestration graphic input either appears as if being drawn by hand in real-time or it may appear in its entirety all at once. Also, optionally, the information may be provided to the 3D display **303** in such a fashion that the 3D telestration graphic input either fades after a time by either disappearing gradually from one end to the other, or by fading all points together. In addition, as previously described, a confidence measure may be computed such as a correlation value, and the brightness of the displayed telestration graphic input may be proportional to the magnitude of the confidence measure.

**FIG. 8** illustrates, as an example, a flow diagram detailing tasks executed by the rendering unit **404** and the graphics overlay module **405** in performing the anatomic structure tracking function **605** of the method described in reference to **FIG. 6****.** In **801,** the rendering unit **404** performs a frame-to-frame (F/F) image correlation by causing the image correlation module **402** to: (a) correlate the most recently captured right camera view with the just prior captured right camera view by the image acquisition module **401,** and (b) correlate the most recently captured left camera view with the just prior captured left camera view by the image acquisition module **401.** By performing this F/F image correlation, a new position in the 3D space of the stereoscopic endoscope is determined for the anatomic structure upon which the telestration graphic input is to be overlayed.

Since the anatomical structure being viewed at the surgical site is only expected to move slowly, if at all, relative to the stereoscopic endoscope **301,** the F/F image correlation performed in **801** may be performed more rapidly than the image correlation performed in **602** to construct the disparity map, since the area over which the image correlation is performed may be reduced. This reduction in area is particularly useful, because unlike the disparity map determination in which the positions of identifying characteristics in the right and left camera views are expected to only differ by their disparity values along horizontal epipolar lines, for anatomy tracking purposes, it is also useful to consider vertical and depth movements.

In **802,** the rendering unit **404** next updates the position of the telestration graphic input in the most recently captured right and left camera views so as to track movement of the anatomic structure upon which it is to be overlayed. In particular, for each shared (i.e., overlayed) point of the anatomic structure and the telestration graphic input, the telestration graphic input point is moved to the new position of its corresponding anatomic structure point in both the right and left camera views, as determined through the F/F image correlation.

Although the various aspects of the present invention have been described with respect to a preferred embodiment, it will be understood that the invention is entitled to full protection within the full scope of the appended claims.

## Claims

1. A method for telestrating on a three-dimensional view of an anatomical structure generated from a pair of stereoscopic images of the anatomical structure, the method including the step of:
receiving a telestration graphic input generated by a user interacting with a two-dimensional touch screen (313, 412);
**characterized in that** the two-dimensional touch screen (313, 412) is displaying one of the pair of stereoscopic images of the anatomical structure, and
**in that** the method includes the steps of:
determining a corresponding telestration graphic input in the other of the pair of stereoscopic images by using a disparity map associated with the pair of stereoscopic images on selected points of the received telestration graphic input so that a three-dimensional view of the telestration graphic input is displayable on a three-dimensional display (303); and
displaying the three-dimensional view of the telestration graphic input as an overlay to the three-dimensional view of the anatomical structure on the three-dimensional display (303).

2. The method according to claim 1, further comprising:
generating the disparity map by using the pair of stereoscopic images.

3. The method according to claim 2, further comprising:
correlating the information for the pair of stereoscopic images with information for a subsequent in time pair of stereoscopic images so as to determine movement of the anatomical structure relative thereto; and
positioning the three-dimensional view of the telestration graphic input so as to track the movement of the anatomical structure.

4. A medical robotic system (100) providing three-dimensional telestration, the system comprising a stereoscopic endoscope (301) configured to generate a pair of stereoscopic images of an anatomical structure, a two-dimensional touch screen (313, 412), a surgeon computer (302) configured to receive a telestration graphic input generated from user interaction with the two-dimensional touch screen (313, 412), and a three-dimensional display (303) for displaying a three-dimensional view of the anatomical structure generated from the pair of stereoscopic images of the anatomical structure, wherein the system is **characterized in that**:
the two-dimensional touch screen (313, 412) is configured to receive one of the pair of stereoscopic images captured by the stereoscopic endoscope (301), and
the surgeon computer (302) is configured to: receive a telestration graphic input generated from user interaction with the two-dimensional touch screen (313, 412); determine a corresponding telestration graphic input for the other of the pair of stereoscopic images by using a disparity map associated with the pair of stereoscopic images on selected points of the received telestration graphic input so that a three-dimensional view of the telestration graphic input is displayable on the three-dimensional display (303); and cause the three-dimensional view of the telestration graphic input to be displayed as an overlay to the three-dimensional view of the anatomical structure on the three-dimensional display (303).

5. The medical robotic system (100) according to claim 4, further comprising:
an expert computer (312) coupled to the two-dimensional touch screen (313); and
interface means (306, 316) for transmitting information for the one of the pair of stereoscopic images to the expert computer (312) prior to receiving the telestration graphic input generated by the user interaction with the two-dimensional touch screen (313).

6. The medical robotic system (100) according to claim 5, wherein the surgeon computer (302) is configured to receive information for the pair of stereoscopic images prior to the interface means (306, 316) transmitting the information for the one of the pair of stereoscopic images to the expert computer (312).

7. The medical robotic system (100) according to claim 6, wherein the surgeon computer (302) is configured to generate the disparity map from the received information for the pair of stereoscopic images of the anatomical structure.

8. The medical robotic system (100) according to claim 6, wherein the surgeon computer (302) is configured to: receive information for a subsequent in time pair of stereoscopic images after the interface means (306, 316) transmits the information for the one of the pair of stereoscopic images to the expert computer (312); correlate the information for the pair of stereoscopic images with the information for the subsequent in time pair of stereoscopic images so as to determine movement of the anatomical structure relative thereto; and position the three-dimensional view of the telestration graphic input so as to track the movement of the anatomical structure.

9. The medical robotic system (100) according to claim 8, wherein the surgeon computer (302) is configured to determine a correlation value from the correlation of the information for the pair of stereoscopic images with the information for the subsequent in time pair of stereoscopic images; and display the telestration graphic input with a brightness proportional to a magnitude of the correlation value.

10. The medical robotic system (100) according to claim 4, wherein the surgeon computer (302) is configured to cause the three-dimensional view of the telestration graphic to be displayed on the three-dimensional display (303) such that the three-dimensional view of the telestration graphic input fades over time.

## Patentansprüche

1. Verfahren zum Telestrieren einer anatomischen Struktur auf einer dreidimensionalen Ansicht, erzeugt von einem Paar stereoskopischen Bildern der anatomischen Struktur, wobei das Verfahren die folgenden Schritte beinhaltet:
Empfangen eines Telestrationsgrafikeingangs, der von einem Benutzer erzeugt wird, der mit einem zweidimensionalen Berührungsbildschirm (313, 412) interagiert;
**dadurch gekennzeichnet, dass** der zweidimensionale Berührungsbildschirm (313, 412) eines aus dem Paar von stereoskopischen Bildern der anatomischen Struktur anzeigt, und dadurch, dass das Verfahren die folgenden Schritte beinhaltet:
Bestimmen eines entsprechenden Telestrationsgrafikeingangs in dem anderen aus dem Paar von stereoskopischen Bildern durch Benutzen einer mit dem Paar von stereoskopischen Bildern assoziierten Disparitätskarte an gewählten Punkten des empfangenen Telestrationsgrafikeingangs, so dass eine dreidimensionale Ansicht des Telestrationsgrafikeingangs auf einem dreidimensionalen Display (303) angezeigt werden kann; und
Anzeigen der dreidimensionalen Ansicht des Telestrationsgrafikeingangs als eine Einblendung auf der dreidimensionalen Ansicht der anatomischen Struktur auf dem dreidimensionalen Display (303).

2. Verfahren nach Anspruch 1, das ferner Folgendes beinhaltet:
Erzeugen der Disparitätskarte mit Hilfe des Paares von stereoskopischen Bildern.

3. Verfahren nach Anspruch 2, das ferner Folgendes beinhaltet:
Korrelieren der Informationen für das Paar von stereoskopischen Bildern mit Informationen für ein zeitlich nachfolgendes Paar von stereoskopischen Bildern, um eine Bewegung der anatomischen Struktur relativ dazu zu bestimmen; und
Positionieren der dreidimensionalen Ansicht des Telestrationsgrafikeingangs, um die Bewegung der anatomischen Struktur zu verfolgen.

4. Medizinisches Robotersystem (100), das dreidimensionale Telestration bereitstellt, wobei das System Folgendes umfasst: ein stereoskopisches Endoskop (301), konfiguriert zum Erzeugen eines Paares von stereoskopischen Bildern einer anatomischen Struktur, einen zweidimensionalen Berührungsbildschirm (313, 412), einen Chirurgencomputer (302), konfiguriert zum Empfangen eines Telestrationsgrafikeingangs, erzeugt von einer Benutzerinteraktion mit dem zweidimensionalen Berührungsbildschirm (313, 412), und ein dreidimensionales Display (303) zum Anzeigen einer dreidimensionalen Ansicht der von dem Paar von stereoskopischen Bildern der anatomischen Struktur erzeugten anatomischen Struktur, wobei das System **dadurch gekennzeichnet ist, dass**:
der zweidimensionale Berührungsbildschirm (313, 412) zum Empfangen von einem aus dem Paar von stereoskopischen Bildern konfiguriert ist, die mit dem stereoskopisches Endoskop (301) erfasst wurden, und
der Chirurgencomputer (302) konfiguriert ist zum: Empfangen eines Telestrationsgrafikeingangs, erzeugt von einer Benutzerinteraktion mit dem zweidimensionalen Berührungsbildschirm (313, 412); Bestimmen eines entsprechenden Telestrationsgrafikeingangs für das andere aus dem Paar von stereoskopischen Bildern anhand einer mit dem Paar von stereoskopischen Bildern assoziierten Disparitätskarte an gewählten Punkten des empfangenen Telestrationsgrafikeingangs, so dass eine dreidimensionale Ansicht des Telestrationsgrafikeingangs auf dem dreidimensionalen Display (303) angezeigt werden kann; und Bewirken, dass die dreidimensionale Ansicht des Telestrationsgrafikeingangs als Einblendung auf der dreidimensionalen Ansicht der anatomischen Struktur auf dem dreidimensionalen Display (303) angezeigt wird.

5. Medizinisches Robotersystem (100) nach Anspruch 4, das ferner Folgendes umfasst:
einen Expertencomputer (312), der mit dem zweidimensionalen Berührungsbildschirm (313) gekoppelt ist; und
Schnittstellenmittel (306, 316) zum Übertragen von Informationen für das eine aus dem Paar von stereoskopischen Bildern zu dem Expertencomputer (312) vor dem Empfangen des durch die Benutzerinteraktion mit dem zweidimensionalen Berührungsbildschirm (313) erzeugten Telestrationsgrafikeingangs.

6. Medizinisches Robotersystem (100) nach Anspruch 5, wobei der Chirurgencomputer (302) zum Empfangen von Informationen für das Paar von stereoskopischen Bildern konfiguriert ist, bevor das Schnittstellenmittel (306, 316) die Informationen für das eine aus dem Paar von stereoskopischen Bildern zum Expertencomputer (312) überträgt.

7. Medizinisches Robotersystem (100) nach Anspruch 6, wobei der Chirurgencomputer (302) zum Erzeugen der Disparitätskarte von den empfangenen Informationen für das Paar von stereoskopischen Bildern der anatomischen Struktur konfiguriert ist.

8. Medizinisches Robotersystem (100) nach Anspruch 6, wobei die Chirurgencomputer (302) konfiguriert ist zum: Empfangen von Informationen für ein zeitlich nachfolgendes Paar von stereoskopischen Bildern, nachdem das Schnittstellenmittel (306, 316) die Informationen für das eine aus dem Paar von stereoskopischen Bildern zum Expertencomputer (312) überträgt; Korrelieren der Informationen für das Paar von stereoskopischen Bildern mit den Informationen für das zeitlich nachfolgende Paar von stereoskopischen Bildern, um eine Bewegung der anatomischen Struktur relativ dazu zu bestimmen; und Positionieren der dreidimensionalen Ansicht des Telestrationsgrafikeingangs, um die Bewegung der anatomischen Struktur zu verfolgen.

9. Medizinisches Robotersystem (100) nach Anspruch 8, wobei der Chirurgencomputer (302) zum Bestimmen eines Korrelationswertes von der Korrelation der Informationen für das Paar von stereoskopischen Bildern mit den Informationen für das zeitlich nachfolgende Paar von stereoskopischen Bildern zu bestimmen; und Anzeigen des Telestrationsgrafikeingangs mit einer Helligkeit proportional zu einer Größe des Korrelationswertes.

10. Medizinisches Robotersystem (100) nach Anspruch 4, wobei der Chirurgencomputer (302) konfiguriert ist um zu bewirken, dass die dreidimensionale Ansicht der Telestrationsgrafik auf dem dreidimensionalen Display (303) angezeigt wird, so dass die dreidimensionale Ansicht des Telestrationsgrafikeingangs im Laufe der Zeit verblasst.

## Revendications

1. Procédé servant à des fins de télé-illustration sur une vue tridimensionnelle d'une structure anatomique générée à partir d'une paire d'images stéréoscopiques de la structure anatomique, le procédé comprenant les étapes consistant à :
recevoir une entrée graphique de télé-illustration générée par un utilisateur en interaction avec un écran tactile bidimensionnel (313, 412) ;
**caractérisé en ce que** l'écran tactile bidimensionnel (313, 412) affiche l'une de la paire d'images stéréoscopiques de la structure anatomique, et
**en ce que** le procédé comprend les étapes consistant à :
déterminer une entrée graphique de télé-illustration correspondante dans l'autre de la paire d'images stéréoscopiques en utilisant une carte de disparités associée à la paire d'images stéréoscopiques sur des points sélectionnés de l'entrée graphique de télé-illustration reçue de telle sorte qu'une vue tridimensionnelle de l'entrée graphique de télé-illustration est en mesure d'être affichée sur un affichage tridimensionnel (303) ; et
afficher la vue tridimensionnelle de l'entrée graphique de télé-illustration sous la forme d'une superposition sur la vue tridimensionnelle de la structure anatomique sur l'affichage tridimensionnel (303).

2. Procédé selon la revendication 1, comportant par ailleurs l'étape consistant à :
générer la carte de disparités en utilisant la paire d'images stéréoscopiques.

3. Procédé selon la revendication 2, comportant par ailleurs les étapes consistant à :
corréler les informations pour la paire d'images stéréoscopiques par rapport aux informations pour une paire ultérieure dans le temps d'images stéréoscopiques de manière à déterminer tout mouvement de la structure anatomique par rapport à celles-ci ; et
positionner la vue tridimensionnelle de l'entrée graphique de télé-illustration de manière à suivre le mouvement de la structure anatomique.

4. Système robotisé médical (100) fournissant une télé-illustration tridimensionnelle, le système comportant un endoscope stéréoscopique (301) configuré pour générer une paire d'images stéréoscopiques d'une structure anatomique, un écran tactile bidimensionnel (313, 412), un ordinateur de chirurgien (302) configuré pour recevoir une entrée graphique de télé-illustration générée par une interaction de l'utilisateur avec l'écran tactile bidimensionnel (313, 412), et un affichage tridimensionnel (303) servant à des fins d'affichage d'une vue tridimensionnelle de la structure anatomique générée à partir de la paire d'images stéréoscopiques de la structure anatomique, dans lequel le système est **caractérisé en ce que** :
l'écran tactile bidimensionnel (313, 412) est configuré pour recevoir l'une de la paire d'images stéréoscopiques capturées par l'endoscope stéréoscopique (301), et
l'ordinateur de chirurgien (302) est configuré pour : recevoir une entrée graphique de télé-illustration générée par l'interaction de l'utilisateur avec l'écran tactile bidimensionnel (313, 412) ; déterminer une entrée graphique de télé-illustration correspondante pour l'autre de la paire d'images stéréoscopiques en utilisant une carte de disparités associée à la paire d'images stéréoscopiques sur des points sélectionnés de l'entrée graphique de télé-illustration reçue de telle sorte qu'une vue tridimensionnelle de l'entrée graphique de télé-illustration est en mesure d'être affichée sur l'affichage tridimensionnel (303) ; et amener la vue tridimensionnelle de l'entrée graphique de télé-illustration à être affichée sous la forme d'une superposition sur la vue tridimensionnelle de la structure anatomique sur l'affichage tridimensionnel (303).

5. Système robotisé médical (100) selon la revendication 4, comportant par ailleurs :
un ordinateur d'expert (312) accouplé à l'écran tactile bidimensionnel (313) ; et
un moyen d'interface (306, 316) servant à transmettre des informations pour ladite l'une de la paire d'images stéréoscopiques à l'ordinateur d'expert (312) avant de recevoir l'entrée graphique de télé-illustration générée par l'interaction de l'utilisateur avec l'écran tactile bidimensionnel (313).

6. Système robotisé médical (100) selon la revendication 5, dans lequel l'ordinateur de chirurgien (302) est configuré pour recevoir des informations pour la paire d'images stéréoscopiques avant que le moyen d'interface (306, 316) ne transmette les informations pour ladite l'une de la paire d'images stéréoscopiques à l'ordinateur d'expert (312).

7. Système robotisé médical (100) selon la revendication 6, dans lequel l'ordinateur de chirurgien (302) est configuré pour générer la carte de disparités à partir des informations reçues pour la paire d'images stéréoscopiques de la structure anatomique.

8. Système robotisé médical (100) selon la revendication 6, dans lequel l'ordinateur de chirurgien (302) est configuré pour : recevoir des informations pour une paire ultérieure dans le temps d'images stéréoscopiques après que le moyen d'interface (306, 316) a transmis les informations pour ladite l'une de la paire d'images stéréoscopiques à l'ordinateur d'expert (312) ; corréler les informations pour la paire d'images stéréoscopiques par rapport aux informations pour la paire ultérieure dans le temps d'images stéréoscopiques de manière à déterminer tout mouvement de la structure anatomique par rapport à celles-ci ; et positionner la vue tridimensionnelle de l'entrée graphique de télé-illustration de manière à suivre le mouvement de la structure anatomique.

9. Système robotisé médical (100) selon la revendication 8, dans lequel l'ordinateur de chirurgien (302) est configuré pour déterminer une valeur de corrélation à partir de la corrélation des informations pour la paire d'images stéréoscopiques par rapport aux informations pour la paire ultérieure dans le temps d'images stéréoscopiques ; et afficher l'entrée graphique de télé-illustration avec une luminosité proportionnelle à une grandeur de la valeur de corrélation.

10. Système robotisé médical (100) selon la revendication 4, dans lequel l'ordinateur de chirurgien (302) est configuré pour amener la vue tridimensionnelle du graphique de télé-illustration à être affichée sur l'affichage tridimensionnel (303) de telle sorte que la vue tridimensionnelle de l'entrée graphique de télé-illustration s'atténue avec le temps.
